Europäisches Patentamt

European Patent Office

Office européen des brevets

(19)

(11) Publication number: **0 007 189**

**B1**

(12)     **EUROPEAN PATENT SPECIFICATION**

(45) Date of publication of patent specification: **02.06.82**

(51) Int. Cl.³: **A 61 N 1/36**

(21) Application number: **79301200.6**

(22) Date of filing: **21.06.79**

(54) Physiologically adaptive cardiac pacemaker.

(30) Priority: **23.06.78 GB 2769278**
**23.06.78 GB 2769378**

(43) Date of publication of application:
**23.01.80 Bulletin 80/2**

(45) Publication of the grant of the patent:
**02.06.82 Bulletin 82/22**

(84) Designated Contracting States:
**AT BE CH DE FR GB IT LU NL SE**

(56) References cited:
**BE - A - 861 850**
**DE - A - 2 252 312**
**FR - A - 2 169 507**
**FR - A - 2 262 958**
**FR - A - 2 311 528**
**FR - A - 2 342 721**
**US - A - 4 091 818**

(73) Proprietor: **CREDIT DU NORD INTERNATIONAL
N.V. (VITAFIN N.V.)
Willemstad Curacao, Netherlands Antilles (NL)**

(72) Inventor: **Rickards, Anthony Francis
20 Quickswood Chalcot Park
London NW3 (GB)**

(74) Representative: **Batchellor, John Robert et al,
BATCHELLOR, KIRK & EYLES 2 Pear Tree Court
Farringdon Road
London EC1R 0DS (GB)**

Physiologically adaptive cardiac pacemaker

This invention relates to a cardiac pacemaker for delivering electrical stimuli to the heart for the purpose of stimulating the heart to react electrically and thus contract mechanically. A pacemaker consists of an electronic generator connected through an electrode to the ventricular myocardium of the heart; the electrode may be unipolar or bipolar.

Although fixed rate generators are known which provide electrical stimuli to the heart at fixed and preset intervals, the present invention is concerned with a pacemaker having a demand generator, which is sensitive to the electrical activity of the heart. The demand generator includes a timing circuit which, unless disabled, causes a stimuli to be delivered at the end of an escape interval. If there is no intrinsic, or naturally occurring, heart activity, the ventricles are electrically inactive and the demand generator acts as a fixed rate generator and delivers pacing stimuli to the heart. If, however, the generator senses an intrinsic heart activity during an escape interval, then the timing circuit is restarted without causing a stimulus, while, if no ventricular activity is sensed during the escape interval, a stimulus is delivered by the generator at the end of that interval and the subsequent pacing interval between consecutive stimuli may be identical, or different. Thus, a demand generator delivers stimuli designed to depolarise the heart only if the natural ventricular rate falls below a preset value corresponding to the escape interval. The demand, or stimulus signal, generator may include modifiable means for setting the escape interval, under the control of the physician for example.

With a healthy heart, the natural rate of ventricular activity responds to nervous and humoral stimuli; in the normal individual, exercise results in an increasing heart rate, which is accomplished by nervous and humoral stimuli increasing the rate at which the natural pacemaker of the heart — the sinus node — depolarises. A pacemaker having a demand generator as described above is incapable of responding to physiological conditions which, in the normal individual, would cause an increase in heart rate. While there have been proposals to cause the pacemaker to react to the physiological conditions of the individual, e.g. atrial activity, tissue pH or respiratory rate, all those proposals have required detectors which are additional to the pacemaker and which sense the required condition.

Thus, French Patent No. 2342721 discloses a pacemaker having circuitry which allows the time between the detection of an atrial contraction and the provision of an electrical stimulus to cause a ventricular contraction, to vary with the rate of the sensed atrial contractions. In other words, the atrial-ventricular delay is adapted to correspond to the sensed atrial rate. German Patent No. 2252312 discloses a demand pacer which contains circuitry for adjusting the escape interval corresponding to the detected heart-beat rate. When there is a failure to detect a natural heart-beat, the pacer paces initially at a rate near the tracked rate, and gradually returns the pacing rate to a predetermined fixed value.

The present invention is based on our realisation that the period of ventricular repolarisation — the interval between the onset of ventricular depolarisation (the QRS complex) and repolarisation (the T wave) — decreases with increase in heart rate, due to the action of hormones released into the blood stream with cardiac effects. In the present invention, the interval between a pacing stimulus delivered by a pacemaker generator and the evoked repolarisation sets the escape interval of the generator for the subsequent stimulus, and thus controls the heart rate.

The present invention thus relates to a physiologically adaptive cardiac pacemaker having circuitry for carrying out demand pacer functions, connectable with an electrode for delivering stimulus signals to a patient's heart and for receiving from the patient's heart signals representative of heart activity, and including stimulus signal generator means for generating and delivering stimulus signals, and modifiable means for setting the escape interval of the stimulus signal generator, and is characterised in the provision of T wave sensing means for sensing a T wave portion of an evoked heart response following a delivered stimulus signal; interval means for determining the time relationship of the T wave relative to the delivered stimulus signal; and modifying means connected to the interval means and the modifiable means for modifying the escape interval as a function of said time relationship.

The invention has the merit that the pacemaker can vary the induced heart rate in accordance with the body requirements using signals sensed from the pacemaker itself, and without the use of special detectors for sensing conditions elsewhere in the body.

So-called "programmable pacemakers" are known which can respond to external instructions given by the physician to modify the energy output of each stimulus, the aim being to set that output above a threshold value of energy below which cardiac electrical activity or depolarisation does not consistently occur in response to the pacing stimuli. The energy output can be modified by adjustment of output voltage, stimulus duration, or current, or a combination of two or more of those parameters.

Due to changes in the patient's condition or due to missetting, the energy output set by the physician may be below the threshold with consequential danger to the patient. Equally, the energy output may be set so far above the threshold that wastage of battery energy and reduction of battery life unnecessarily result.

Advantageously, the pacemaker may additionally include means for testing for the threshold of said patient to said stimulus signals, said testing means including said T wave sensing means and also means for increasing the intensity of the next stimulus signal following the absence of an evoked T wave. That arrangement, when provided, ensures that, if the energy output is initially set below the threshold, or if the threshold rises, the energy output is automatically raised until the threshold is exceeded.

Preferably the energy output is adjustable in steps and the adjusting circuit is arranged to increase the energy output by one step each time the absence of an evoked response is detected.

The invention may optionally make provision for automatically setting the energy output above, or as close as possible to, the threshold. Thus, the pacemaker preferably further includes an externally operable circuit, which when rendered operative, decreases the energy output after each stimulus until the adjusting circuit detects the absence of an evoked response. When that absence is detected indicating that the energy output is below the threshold, the adjusting circuit increases the energy output as described to above the threshold.

The invention will be more readily understood by way of example from the following description of a cardiac pacemaker in accordance therewith, reference being made to the accompanying drawings, in which:—

Figure 1 is a block schematic circuit diagram of the pacemaker;

Figures 2a, b and c are traces explaining the operation of one part of the pacemaker's circuit; and

Figure 3 explains the operation of a second part of the circuit.

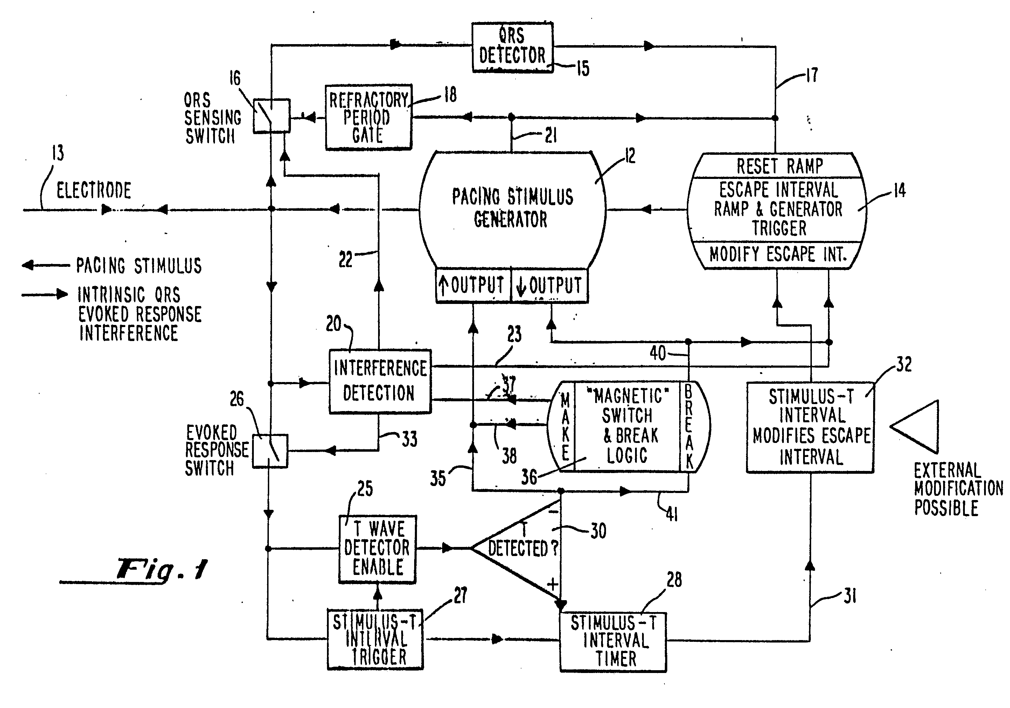

The pacemaker circuit shown in Figure 1 consists of a generator circuit 12, which emits a stimulus pulse on the electrode 13, when triggered by a trigger circuit 14. The trigger circuit 14 generates a ramp voltage with an adjustable rate of rise and, when the voltage exceeds a fixed value emits a trigger pulse to trigger the generator circuit 12.

QRS detector 15 is connected through a switch 16 to the electrode and is capable of detecting the spontaneous QRS complex of an intrinsic heart activity. When the complex is detected, a signal is passed on line 17 to reset the trigger circuit 14. The switch 16, which can be an electronic gate, is controlled, firstly, by a refractory period gate 18 and, secondly, by an interference detector 20. When the generator circuit 12 emits a stimulus to the electrode 13, a signal is simultaneously emitted on line 21 to reset the trigger circuit 14 and to operate gate 18 which opens switch 16 for a predetermined period which is greater than the interval between the delivery of a stimulus by generator circuit 12 and the QRS complex of the evoked response. The detector 20 detects external electromagnetic interference and, on the occurrence of such detected interference picked up on the electrode 13, opens the switch 16, thereby disabling the detector 15. Simultaneously, detector 20 passes on line 23 a signal to control the trigger 14.

The circuit so far described is already known and operates as follows: the trigger circuit 14 is set to give an escape interval corresponding to the lowest desired pacing rate. The escape interval is the interval needed for the ramp voltage to reach the fixed value after being reset. During intrinsic heart activity, the generator circuit 12 is quiescent, because the arrival of each QRS complex is detected by detector 15 and the trigger circuit 14 is reset before the ramp voltage reaches the fixed value and causes the generator circuit 12 to be triggered. If an intrinsic QRS complex is not detected within the escape interval, the generator circuit 12 is triggered and emits a stimulus to the electrode 13, and simultaneously resets the trigger 14 to initiate the next escape interval, and opens switch 16 for a period sufficiently long to prevent the detector 15 receiving the evoked response of the stimulus.

If an intrinsic QRS complex arrives before the end of the next escape interval, the trigger circuit 14 is reset, so that no stimulus is generated by the generating circuit 12. If, however, no QRS complex is detected within that escape interval, the generator 12 is triggered and produces a stimulus, as before.

If the interference detector 20 detects the presence of external electromagnetic interference, switch 16 is opened to disable detector 15 for so long as the interference subsists. Simultaneously, the detector 20 causes the rate of the ramp voltage generated by the trigger circuit 14 to be modified, in order to decrease the escape interval to a predetermined rate (say 90 per minute) which may or may not be faster than that corresponding to the normal escape interval of the circuit. The consequence is that, so long as the interference continues, pacing stimuli are emitted to electrode 13 at a fixed rate, regardless of intrinsic heart activity.

In order that the pacemaker should react to physiological changes, the pacemaker circuit as described above is modified as follows: a T wave detector 25 is connected to the electrode 13 through an evoked response switch 26 and is designed to detect ventricular repolarisation. Detector 25 is normally disabled, but can be enabled by a trigger circuit 27, which is also connected to switch 26 and which can be energised only by a stimulus emitted by generating circuit 12, and not by an intrinsic QRS complex. When energised, trigger 27 emits after a short predetermined interval an enabling signal to T wave detector 25. The stimulus pulse is also applied by trigger circuit 27 to a timing circuit 28, but without a delay; timing circuit 28 is then set in operation. When detector 25 detects a T wave, it passes a pulse to logic circuit 30, which detects the presence or absence of a T wave. If a T wave is detected, the pulse is applied to timing circuit 28 which ceases its timing operation and emits on line 31 a signal representing the measured time interval which is the interval between the receipt of the stimulus and the receipt of the T wave. The signal is applied to a modifying circuit 32 which modifies the escape interval of trigger circuit 14, by altering the rate at which the ramp voltage increases. If the stimulus-T wave interval, as measured by timing circuit 28 decreases, the escape interval is also decreased, but not necessarily proportionally.

If, then, the physiological conditions of the patient alters to require a faster rate of pacing, the physiological change is detected by a reduction in the stimulus-T wave interval. The resulting decrease in the escape interval of trigger circuit 14 causes the generating circuit 12 to deliver stimuli at a higher rate. If an intrinsic QRS activity is at any time detected by detector 15, the trigger circuit 14 is reset as before, and T wave detector 25 will not be enabled, since trigger circuit 27 is operated only by stimuli, with the result that the escape interval modification circuit is inoperative. If external electromagnetic interference is present, the interference detector 20 opens the evoked response switch 26 through line 33 and the pacemaker returns to the fixed rate pacing mode described above in relation to interference detection.

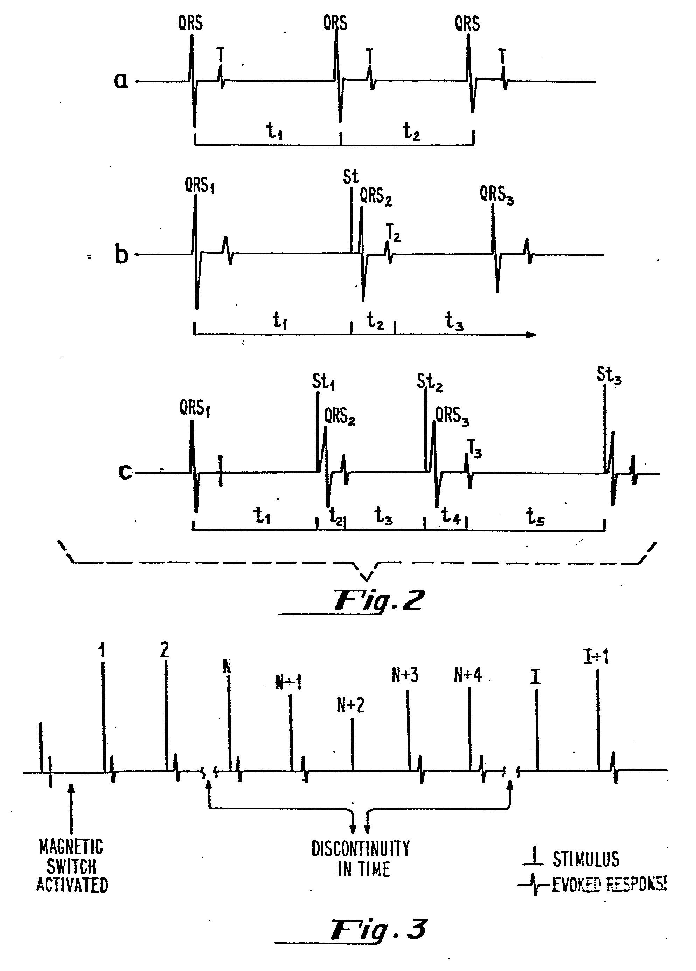

The operation of the circuit just described for varying the escape interval is illustrated in Figure 2. The following notes relate to Figure 2:

a. Times $t_1$ and $t_2$ do not exceed the escape interval of the demand generator (which senses the QRS complexes) and is therefore inhibited from pacing.

b. Time $t_1$ is greater than the escape interval of the generator and a pacing stimulus (St) is delivered which evokes a QRST complex. The St to T wave interval $(t_2)$ is measured and an escape interval $t_3$ is set. A naturally occurring QRST complex occurs before $t_3$ expires and inhibits the generator.

c. Time $t_1$ exceeds the escape interval of the generator and thus $St_1$ is delivered. The interval from stimulus to T wave is detected $(t_2)$ and $t_3$ — the generator escape interval set. Following stimulus 2 $(St_2)$ the stimulus T interval is again sensed and the subsequent escape interval $(t_5)$ set.

Thus the escape intervals following paced beats $(t_3$ and $t_5)$ are a function of the stimulus-T intervals $(t_2$ and $t_4)$ of the previous beats such that decreases in stimulus T intervals produce decreases in escape intervals and thus increased rate.

Figure 2a represents the condition where intrinsic QRST complexes are detected. Provided that the times $t_1$ and $t_2$ between successive QRS complexes detected by detector 15 are less than the escape interval of the trigger circuit 14, there is no pacing stimulus generated. In Figure 2b, no subsequent QRS complex is detected by detector 15 within the escape interval of the generator and, therefore, a stimulus St is delivered to the electrode 13 and therefore to the heart, producing an evoked response — $QRS_2$. The time interval $t_2$ from the stimulus St to the T wave of the evoked response is measured by timing circuit 28 and the escape interval $t_3$ of the trigger circuit 14 is modified appropriately. A naturally occuring complex $QRS_3$ occurring before the end of the new escape interval $t_3$ inhibits the pacing generator 12.

Figure 2c demonstrates the situation where a naturally occurring QRS complex is not detected by detector 15 in the new escape interval $t_3$. In that case, a second stimulus $St_2$ is generated and, as before, the interval $t_4$ between stimulus $St_2$ and the T wave — $T_3$ of the evoked response is measured and sets the escape interval for the next period. Figure 2c shows that $t_4$ is considerably longer than $t_2$, and therefore the new escape interval $t_5$ is again increased. The figure also shows the absence of a detected QRS complex in the new escape interval $t_5$ and therefore the production of a further stimulus $St_3$.

The voltage level of the stimulus generated by generating circuit 12 must of course be greater than the threshold — the value of the voltage below which cardiac electric activity or depolarisation does not consistently occur in response to the pacing stimuli. On the other hand, if the voltage level is too far above the threshold, wastage of battery energy occurs, requiring early battery replacement. The circuit of Figure 1 includes circuit elements designed to maintain the stimulus energy level (particularly the stimulus voltage) voltage at a minimum level, but above the threshold.

Reverting to the logic circuit 30, the absence of a T wave following a stimulus is detected by circuits 25, 27 and 30 to cause a signal to be applied on line 35 to the stimulus generating circuit 12 and to increase the stimulus output level by one step. Thus, if the voltage level is initially as 25% of the maximum level, the generating circuit 12 is increased in successive cycles to 50%, 75% and 100% of the maximum, provided that no evoked response T wave is produced in those cycles.

The circuit includes a magnetic switch 36, usually a reed switch, activated externally by the physician. When the switch is activated to the "make" condition, it causes through line 37 the interference detector 20 to be switched on, causing the generating circuit 12 to emit stimuli continuously at a fixed rate as described above. Also, the magnetic switch applies through lines 38 and 35 a signal to the generating circuit 12 to cause the output stimulus level to be increased, as described, to the maximum value.

When the magnet is removed and the magnetic switch returns to the "break" condition a signal is applied on line 40 to the generating circuit 12, to cause that circuit to reduce the output level in a sequence of steps in successive cycles. Also when the magnetic switch 36 is in the "break" condition, interference detector 20 is deenergised, causing T wave detector 25 to be operative, and the trigger circuit 14 receives the signal on line 40 to maintain the escape interval at a rate which may or may not be higher than the basic escape interval of trigger 14. In this way, the output level of the generating circuit 12 is reduced in steps, as long as detector 25 detects a T wave. As soon as the T wave detector 25 fails to detect a T wave, showing that the stimulus level is below the threshold, the resulting signal from logic circuit 30 is applied on line 41 to prevent further reduction in the energy level, and on line 35 to increase the level again by one step. If the absence of T wave is not detected, reduction of the output level of generating circuit 12 stops at a predetermined minimum of, say 25% of the maximum.

The operation is illustrated diagrammatically in Figure 3. The following notes relate to Figure 3:

Typical sequence following magnet switch activation:

Stimulus 1, 2 following magnet switch is at maximum output and remains there for testing purposes for N − 1 beats.

Stimulus N at say 75% output evokes a response.

Stimulus N + 1 at say 50% output evokes a response.

Stimulus N + 2 at say 25% output does not evoke a response.

Stimulus N + 3 increases to 50% output — response evoked.

Typical "naturally" occurring sequence:

Stimulus I of say 50% does not evoke a response.

Stimulus I + 1 increases to 75% output and evokes a response.

Figure 3 assumes that magnetic switch 36 is made at a time X. Generating circuit 12 is instructed to increase the output level to the maximum as shown at 1; that level is retained for test purposes for a further N − 2 beats. The magnetic switch 36 is then brought to the "break" condition after the (N − 1) beat, so that for the Nth beat the level is reduced to 75% of the maximum. Similarly, the level for the N + 1 beat is 50% and that for the N + 2 beat 25%. The 25% level of the N + 2 beat is below the threshold and the resulting absence of a T wave is detected by logic circuit 30 to cause the level for the N + 3 beat to be 50%, which produces an evoked response. The output level is thus retained at 50% for the N + 4 beat and subsequent beats, unless of course the absence of a T wave is subsequently detected. If stimulus I does not produce an evoked response, that fact is detected by a logic circuit 30 and the output level of the stimulus generator is increased for stimulus I + 1 to the 75% value.

It will therefore be seen that only the physician controlling the magnetic switch 36 can cause a reduction in stimulus output level, whereas the circuit automatically operates to increase the level if there should be no evoked response following a stimulus. In this way, the output level is maintained at that step immediately above the threshold and battery energy economy is achieved.

## Claims

1. A physiologically adaptive cardiac pacemaker having circuitry for carrying out demand pacer functions, connectable with an electrode (13) for delivering stimulus signals to a patient's heart and for receiving from the patient's heart signals representative of heart activity, and including stimulus signal generator means for generating and delivering stimulus signals, and modifiable means (14) for setting the escape interval of the stimulus signal generator characterised in the provision of T wave sensing means (25, 27, 30) for sensing a T wave portion of an evoked heart response following a delivered stimulus signal; interval means (28) for determining the time relationship of the T wave relative to the delivered stimulus signal; and modifying means (32) connected to the interval means and the modifiable means for modifying the escape interval as a function of said time relationship.

2. The cardiac pacemaker as claimed in Claim 1, wherein said T wave sensing means comprises stimulus detection means (27) for detecting when a stimulus signal has been delivered and for enabling said T wave sensing means only upon detecting that a stimulus signal has been delivered.

3. The cardiac pacemaker as claimed in Claim 2, wherein said stimulus detection means (27) enables said T wave sensing means for only a limited time period following detection of a delivered stimulus signal.

4. The cardiac pacemaker as claimed in Claim 3, wherein said stimulus detection means initiates said limited time period after a predetermined time delay following detection of a delivered stimulus signal.

5. The cardiac pacemaker as claimed in any one of the preceding claims, including means (20) for detecting the presence of electromagnetic interference and means (26) for operatively disconnecting said T wave sensing means when said interference is detected to be present.

6. The cardiac pacemaker as claimed in Claim 1, wherein said interval means (28) comprises a circuit for measuring the stimulus-T wave interval and for generating a signal representative of said measured interval.

7. The cardiac pacemaker as claimed in Claim 6, wherein said modifying means (32) has means for rendering it operative only following delivered stimulus signals, and said stimulus signal generator means (12, 14) has a predetermined normal escape interval at which it operates when not modified by said modifying means, such that the pacer escape interval is said normal escape interval following natural heartbeats.

8. The cardiac pacemaker as claimed in Claim 7, wherein said modifying means (32) is adapted for external modification of said function.

9. The cardiac pacemaker as claimed in Claim 6, wherein said modifying means (32) modifies said escape interval in the same direction as said measured interval.

10. The cardiac pacemaker as claimed in Claim 1, comprising a common electrode connection means for connecting stimulus signals and detected heart signals to said T wave sensing means.

11. The cardiac pacemaker as claimed in Claim 1, wherein the interval means (28) determines the time interval between each delivered stimulus and the resulting T wave, and the modifying means (32) controls the modifiable means (14) to set the escape interval as a function of a determined time interval.

12. The cardiac pacemaker as claimed in any one of the preceding claims, in combination with said electrode (13), which electrode is adapted to be placed in the ventricle for delivering pacing stimuli and for detecting QRS waves and T waves.

13. The cardiac pacemaker as claimed in any one of the preceding claims, wherein said stimulus generator means (12, 14) includes circuitry (14) for generating a ramp voltage with an adjustable rate of rise, and means for triggering generation of a pacing stimulus when said ramp voltage exceeds a fixed value.

14. The cardiac pacemaker as claimed in any one of the preceding claims, and comprising a QRS wave detecting circuit, characterised in that the T wave sensing means (25, 27, 30) and the QRS wave detecting circuit (15) are connected to a common point wherein said heart activity signals are received from the electrode.

15. The cardiac pacemaker as claimed in any one of the preceding claims, comprising means (30) connected to said T wave sensing means and to said stimulus signal generator means for determining when there is or is not an evoked T wave following a stimulus pulse, and means (35, 12) controlling said stimulus signal generator means for causing it to produce a stimulating pulse of increased energy following detection of the absence of an evoked T wave.

16. The cardiac pacemaker as claimed in Claim 15, wherein said stimulus signal generator means (12) comprises amplitude means for setting the amplitude of said stimulus signals, and control means for controlling said amplitude means to increase pulse amplitude following sensing of the absence of a T wave following a delivered pacing pulse.

17. The cardiac pacemaker as claimed in any one of claims 1 to 14, comprising means for testing for the threshold of said patient to said stimulus signals, said testing means including said T wave sensing means and also means for increasing the intensity of the next stimulus signal following the absence of an evoked T wave.

## Revendications

1. Stimulateur cardiaque à adaptation physiologique comportant un montage pour l'exécution des fonctions de stimulation, pouvant être relié à une électrode (13) destinée à délivrer des signaux de stimulation au coeur d'un patient et pour recevoir de ce coeur des signaux représentatifs de l'activité cardiaque, et incluant un générateur pour produire et délivrer des signaux de stimulation, ainsi que des moyens modifiables (14) pour régler l'intervalle de rétablissement du générateur de signaux de stimulation caractérisé en ce qu'il comprend des moyens (25, 27, 30) pour détecter l'onde T résultant d'une réponse cardiaque stimulée faisant suite à la délivrance d'un signal de stimulation, des moyens chronométriques (28) pour déterminer la relation temporelle entre l'onde T et le signal de stimulation correspondant et des moyens de modification (32) connectés aux moyens chronométriques et aux moyens modifiables pour modifier l'intervalle de rétablissement en fonction de cette relation temporelle.

2. Stimulateur cardiaque selon la revendication 1, caractérisé en ce que les moyens de détection de l'onde T comprennent des moyens (27) pour détecter quand un signal de stimulation a été délivré et pour activer lesdits moyens de détection de l'onde T seulement après que la délivrance d'un signal de stimulation a été détectée.

3. Stimulateur cardiaque selon la revendication 2, caractérisé en ce que lesdits moyens de détection de stimulation (27) activent lesdits moyens de détection de l'onde T seulement pendant une période de temps limitée faisant suite à la détection d'un signal de stimulation.

4. Stimulateur cardiaque selon la revendication 3, caractérisé en ce que lesdits moyens de détection de stimulation déclenchent ladite période de temps limitée après un délai prédéterminé faisant suite à la détection de la délivrance d'un signal de stimulation.

5. Stimulateur cardiaque selon l'une quelconque des revendications précédentes, caractérisé en ce qu'il comprend des moyens (20) pour détecter la présence d'une interférence électromagnétique et des moyens (26) pour déconnecter fonctionnellement lesdits moyens de détection de l'onde T quand la présence d'une interférence est détectée.

6. Stimulateur cardiaque selon la revendication 1, caractérisé en ce que lesdits moyens chronométriques (28) comprennent un circuit pour mesurer l'intervalle entre le stimulus et l'onde T et pour engendrer un signal représentatif de l'intervalle ainsi mesuré.

7. Stimulateur cardiaque selon la revendication 6, caractérisé en ce que lesdits moyens de modification (32) comprennent des moyens qui font qu'ils n'entrent en action qu'après la délivrance des signaux de stimulation et en ce que ledit générateur de signaux de stimulation (12, 14) comporte un intervalle de rétablissement normal prédéterminé suivant lequel il opère quand il n'a pas été modifié par lesdits moyens de modification, de sorte que l'intervalle de rétablissement du stimulateur est ledit intervalle de rétablissement normal qui suit les battements cardiaques naturels.

8. Stimulateur cardiaque selon la revendication 7 caractérisé en ce que lesdits moyens de modification (32) sont conçus pour permettre de modifier du l'entérieur ladite fonction.

9. Stimulateur cardiaque selon la revendication 6, caractérisé en ce que lesdits moyens de modification modifient ledit intervalle de rétablissement dans la même direction que ledit intervalle mesuré.

10. Stimulateur cardiaque selon la revendication 1, caractérisé en ce qu'il comprend une électrode commune pour transmettre les signaux de stimulation et les signaux cardiaques détectés auxdits moyens de détection de l'onde T.

11. Stimulateur cardiaque selon la revendication 1, caractérisé en ce que lesdits moyens chronométriques (28) déterminent l'intervalle de temps qui s'écoule entre chaque stimulus et l'onde T résultante et lesdits moyens de modification (32) règlent les moyens modifiables (14) pour ajuster ledit intervalle de rétablissement en fonction de l'intervalle de temps ainsi déterminé.

12. Stimulateur cardiaque selon l'une quelconque des revendications précédentes caractérisé en ce qu'il est associé à une électrode (13) qui est adaptée à être placée dans le ventricule du patient pour délivrer des signaux de stimulation cardiaques et pour détecter les ondes QRS et les ondes T.

13. Stimulateur cardiaque selon l'une quelconque des revendications précédentes caractérisé en ce que ledit générateur de signaux de stimulation (12, 14) comporte des circuits (14) pour engendrer une tension en dents de scie ayant une vitesse de croissance ou une "pente" réglable et des moyens pour déclencher la génération d'un stimulus cardiaque quand ladite tension en dents de scie dépasse une valeur déterminée.

14. Stimulateur cardiaque selon l'une quelconque des revendications précédentes et comprenant un circuit de détection des ondes QRS, caractérisé en ce que les moyens de détection de l'onde T (25, 27, 30) et le circuit de détection des ondes QRS (15) sont connectés à un point commun qui reçoit de l'électrode des signaux représentatifs de l'activité cardiaque.

15. Stimulateur cardiaque selon l'une quelconque des revendications précédentes caractérisé en ce qu'il comprend des· moyens (30) connectés auxdits moyens de détection des ondes T et audit générateur de signaux de stimulation pour déterminer la présence ou l'absence d'une onde T en réponse à une impulsion de stimulation et des moyens (35, 12) pour régler le générateur de signaux de stimulation afin qu'ils produisent une impulsion de stimulation ayant une plus grande énergie lorsque l'absence d'une onde T de réponse est constatée.

16. Stimulateur cardiaque selon la revendication 15 caractérisé en ce que ledit générateur de signaux de stimulation (12) comprend des moyens pour régler l'amplitude desdits signaux de stimulation et des moyens pour régler lesdits moyens de réglage d'amplitude de façon à augmenter l'amplitude des impulsions après que l'absence d'une onde T faisant suite à une impulsion de stimulation est constatée.

17. Stimulateur cardiaque selon l'une quelconque des revendications 1 à 14 caractérisé en ce qu'il comprend des moyens pour mesurer le seuil de réponse cardiaque dudit patient auxdits signaux de stimulation, lesdits moyens de mesure incluant lesdits moyens de détection de l'onde T et aussi des moyens pour augmenter l'intensité du signal de stimulation suivant l'absence d'une onde T stimulée.

**Patentansprüche**

1. Physiologisch adaptierender Herzschrittmacher mit einer Schaltung zum Ausführen von bedarfsabhängigen Schrittfunktionen, wobei die Schaltung mit einer Elektrode (13) verbunden ist, die Stimulus-signale an das Herz eines Patienten abgibt und von dem Herz die Herzaktivität kennzeichende Signale empfängt, mit einem Stimulussignal-Generator zur Erzeugung und Abgabe der Stimulussignale, und mit Einstellmitteln (14) zum Festlegen des Abgabeintervalls des Stimulussignal-Generators, da-durch gekennzeichnet, daß eine T-Wellen-Fühleinrichtung (25, 27, 30) vorgesehen ist, die einen T-Wellen-Abschnitt einer evozierten Herzantwort abfühlt, welche einem abgegebenen Stimulussignal nachfolgt, daß eine Intervalleinrichtung (28) vorgesehen ist, welche die Zeitrelation der T-Welle relativ zum abgegebenen Stimulussignal feststellt, und daß eine Änderungseinrichtung (32) mit der Intervalleinrichtung (28) und den Einstellmitteln (14) verbunden ist und das Abgabeintervall als Funktion der festgestellten Zeitrelation ändert.

2. Herzschrittmacher nach Anspruch 1, dadurch gekennzeichnet, daß die T-Welle-Fühleinrichtung (25, 27, 30) einen Stimulusdetektor (27) enthält, der feststellt, wenn ein Stimulussignal abgegeben ist und die T-Wellen-Fühleinrichtung nur aktiviert, nachdem festgestellt wurde, daß ein Stimulussignal abgegeben wurde.

3. Herzschrittmacher nach Anspruch 2, dadurch gekennzeichnet, daß der Stimulusdetektor (27) die T-Wellen-Fühleinrichtung nur für eine begrenzte Zeitperiode aktiviert, die dem Empfang eines abgegebenen Stimulussignals nachfolgt.

4. Herzschrittmacher nach Anspruch 3, dadurch gekennzeichnet, daß der Stimulusdetektor (27) die begrenzte Zeitperiode nach einer vorgegebenen, dem Empfang eines abgegebenen Stimulussignals nachfolgenden Zeitverzögerung auslöst.

5. Herzschrittmacher nach einem der vorstehenden Ansprüche, dadurch gekennzeichnet, daß Einrichtungen (20) vorgesehen sind, welche das Vorhandensein einer elektromagnetischen Interferenz feststellen und daß Einrichtungen (26) vorgesehen sind, welche die T-Wellen-Fühleinrichtung abschalten, wenn elektro-magnetische Interferenz vorhanden ist.

6. Herzschrittmacher nach Anspruch 1, dadurch gekennzeichnet, daß die Intervalleinrichtung (28) eine Schaltung zum Messen des Stimulus/T-Wellen-intervalls, und zur Erzeugung eines das gemessene Intervall kennzeichnende Signale enthält.

7. Herzschrittmacher nach Anspruch 6, dadurch gekennzeichnet, daß die Veränderungseinrichtung (32) Mittel enthält, welche die Veränderungseinrichtung (32) nur nach abgegebenen Stimulussignalen aktivieren, und daß der Stimulussignal-Generator (12, 14) ein vorgegebenes normales Abgabeintervall besitzt, mit dem der Stimulussignal-Generator arbeitet, sofern keine Veränderung durch die Veränderungseinrichtung erfolgt, wobei das Schrittabgabeintervall gleich dem normalen Abgabeintervall ist, das den natürlichen Herzschlägen folgt.

8. Herzschrittmacher nach Anspruch 7, dadurch gekennzeichnet, daß die Veränderungseinrichtung (32) eine externe Veränderung ihrer Funktion erlaubt.

9. Herzschrittmacher nach Anspruch 6, dadurch gekennzeichnet, daß die Veränderungseinrichtung (32) das Abgabeintervall in derselben Richtung wie das gemessene Intervall verändert.

10. Herzschrittmacher nach Anspruch 1, dadurch gekennzeichnet, daß ein gemeinsamer Elektrodenanschluß zum Zuführen der Stimulussignale und der abgefühlten Herzsignale an die T-Wellen-Fühleinrichtung vorgesehen ist.

11. Herzschrittmacher nach Anspruch 1, dadurch gekennzeichnet, daß die Intervalleinrichtung (28) das Zeitintervall zwischen abgegebenem Stimulus und der resultierenden T-Welle feststellt, und daß die Veränderungseinrichtung (32) die Einstelleinrichtung (14) so steuert, daß das Abgabeintervall eine Funktion eines vorgegebenen Zeitintervalls ist.

12. Herzschrittmacher nach einem der vorstehenden Ansprüche, dadurch gekennzeichnet, daß die Elektrode (13) so ausgebildet ist, daß sie in dem Ventrikel angeordnet werden kann und Schrittstimuli abgibt und QRS-Wellen und T-Wellen empfängt.

13. Herzschrittmacher nach einem der vorstehenden Ansprüche, dadurch gekennzeichnet, daß der Stimulussignal-Generator (12, 14) eine Schaltung (14) zur Erzeugung einer Sägezahnspannung mit einstellbarer Flankensteilheit und Mittel zum Triggern und Erzeugen eines Schrittstimulus enthält, wenn die Sägezahnspannung einen festen Wert überschreitet.

14. Herzschrittmacher nach einem der vorstehenden Ansprüche, mit einer QRS-Wellen-Detektorschaltung, dadurch gekennzeichnet, daß die T-Wellen-Fühleinrichtung (25, 27, 30) und die QRS-Wellen-Detektorschaltung (15) an einer gemeinsamen Stelle miteinander verbunden sind, an der die Herzaktivitätssignale von der Elektrode empfangen werden.

15. Herzschrittmacher nach einem der vorstehenden Ansprüche, dadurch gekennzeichnet, daß eine Einrichtung (30) mit der T-Wellen-Fühleinrichtung und dem Stimulussignal-Generator (12, 14) verbunden ist und feststellt, wenn eine evozierte T-Welle im Gefolge eines Stimulusimpulses vorhanden bzw. nicht vorhanden ist, und daß Einrichtungen (35, 12) den Stimulussignal-Generator so steuern, daß er einen Stimulusimpuls erhöhter Energie erzeugt, wenn die Abwesenheit einer evozierten T-Welle festgestellt wird.

16. Herzschrittmacher nach Anspruch 15, dadurch gekennzeichnet, daß der Stimulussignal-Generator eine Amplitudeneinrichtung zum Setzen der Amplitude der Stimulussignale, und eine Steuereinrichtung enthält, um die Amplitudeneinrichtung so zu steuern, daß diese bei Abwesenheit einer T-Welle nach einem abgegebenen Schrittimpuls die Impulsamplitude erhöht.

17. Herzschrittmacher nach einem der Ansprüche 1 bis 14, dadurch gekennzeichnet, daß Einrichtungen zum Prüfen des Pegels des Patienten gegenüber den Stimulussignalen vorgesehen sind, daß diese Testeinrichtung die T-Wellen-Fühleinrichtung und eine weitere Einrichtung enthält, um die Intensität des nächsten Stimulussignales zu erhöhen, welches der Abwesenheit einer evozierten T-Welle nachfolgt.

Fig.1

Fig. 2

MAGNETIC
SWITCH
ACTIVATED

DISCONTINUITY
IN TIME

⊥ STIMULUS
⋀ EVOKED RESPONSE

Fig. 3

2